(19) Europäisches Patentamt
European Patent Office
Office européen des brevets

(11) Veröffentlichungsnummer: **0 056 594**
**B1**

# (12) EUROPÄISCHE PATENTSCHRIFT

(45) Veröffentlichungstag der Patentschrift: **12.09.84**

(51) Int. Cl.³: **C 07 C 103/52,** A 61 K 37/02

(21) Anmeldenummer: **82100127.8**

(22) Anmeldetag: **08.01.82**

(54) **Thymosin-alpha-1-Fragmente enthaltende Arzneimittel mit immunregulierender Wirkung und Thymosin-alpha-1-Fragmente.**

(30) Priorität: **14.01.81 DE 3100974**

(43) Veröffentlichungstag der Anmeldung: **28.07.82 Patentblatt 82/30**

(45) Bekanntmachung des Hinweises auf die Patenterteilung: **12.09.84 Patentblatt 84/37**

(84) Benannte Vertragsstaaten: **BE CH FR GB LI NL**

(56) Entgegenhaltungen:
**EP - A - 0 025 897**
**EP - A - 0 026 464**
**EP - A - 0 033 384**
**EP - A - 0 035 454**
**EP - A - 0 048 573**

**CHEMICAL ABSTRACTS, Band 93, Nr. 25, 22. Dezember 1980, Seite 423, Zusammenfassung 234525x COLUMBUS OHIO (US)**
**BIOCHEMISTRY, Band 19, 1980 R. WETZEL u.a.: "Production of biologically active N alpha-Desacetyl thymosin alpha 1 in Escherichia Coli through Expression of a chemically Synthesized gene" Seiten 6096-6104**
**JOURNAL OF PHARMACEUTICAL SCIENCES, Band 63, Nr. 3 März 1974 B.J. JOHNSON: "Synthesis, Structure and Biological Properties of Sequential Polypeptides"**

(73) Patentinhaber: **Max-Planck-Gesellschaft zur Förderung der Wissenschaften e.V., Bunsenstrasse 10, D-3400 Göttingen (DE)**

(72) Erfinder: **Birr, Christian, Dr., Eichendorffstrasse 31, D-6906 Leimen/St. Ilgen (DE)**
Erfinder: **Ciardelly, Thomas, Dr., 159, Nashua Street, Milford New Hampshire 03055 (US)**

(74) Vertreter: **Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86 (DE)**

(56) Entgegenhaltungen: (Fortsetzung)
**Seiten 313-330**
**CHEMICAL ABSTRACTS, Band 70, Nr. 11, 17. März 1969, Seite 114; Zusammenfassung 45169y COLUMBUS OHIO (US)**
**JUSTUS LIEBIGS: "ANNALEN DER CHEMIE" Band 649 (1961) E. KLIEGER und H. GIBIAN: "Weitere Synthesen von Glutamylpeptiden mit Carbobenzoxy-l-Pyroglutaminsäure", Seiten 183-198**
**CHEMICAL SUBSTANCE INDEX 1972-1976 CHEMICAL ABSTRACTS COLUMBUS OHIO (US)**
**CHEMICAL ABSTRACTS, 8th Coll. SUBJECT INDEX, 1967-1971 COLUMBUS OHIO (US)**
**CHEMICAL PHARMACEUTICAL BULLETIN, Band 27, Nr. 12, 1979 TAKASHIA. u.a.: "The effect of a synthetic thymosin alpha 1 fragment on the inhibition of E-rosette formation by the serum of a patient with nephrotic syndrome" seiten 3171-3175**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 056 594 B1

ACTORUM AG

**Beschreibung**

Die Erfindung betrifft Arzneimittel mit immunregulierender Wirkung, die zur Behandlung von Immunmangelerkrankungen, von Virusinfektionen, des beschleunigten Alterns, Tumorbildungswahrscheinlichkeit und insbesondere von Krebs eingesetzt werden können, sowie Thymosin-$\alpha_1$-Fragmente, die in diesem Arzneimitteln als Wirkstoff enthalten sind.

Thymosin-$\alpha_1$ ist ein saures Polypeptid der Thymusdrüse mit der Sequenz

1 2 3 4 5 6 7 8 9 10 11 12 13 14
Ser-Asp-Ala-Ala-Val-Asp-Thr-Ser-Ser-Glu-Ile-Thr-Thr-Lys-
15 16 17 18 19 20 21 22 23 24 25 26 27 28
Asp-Leu-Lys-Glu-Lys-Lys-Glu-Val-Val-Glu-Glu-Ala-Glu-Asn

in dem die erste Aminosäure, Serin, acetyliert vorliegt.

Thymosin-$\alpha_1$-Präparate finden klinische Anwendung bei der Krebsbekämpfung und im Rahmen der Regulation des immunologischen Abwehrapparates (P. B. Chretienne et. al., „J. D. Cancer treatment Reports", *62*, 1787-1790 (1978)). Da sich Thymosin-$\alpha_1$ nur sehr mühsam aus Thymusdrüsen isolieren lässt, wurden bereits Methoden zu seiner Totalsynthese vorgeschlagen 1-20(J.A.C.S., *101*, 252-254 (1979), DE-OS Nr. 2919592 der Anmelderin). Wie in EP-A Nr. 0033384 offenbart, haben einige Fragmente des Thymosin-$\alpha_1$ bereits die gleichen immunregulierenden Wirkungen wie Thymosin-$\alpha_1$, wenngleich auch in geringerem Ausmass. Trotz der etwas schwächeren Wirksamkeit bietet die Verwendung von Thymosin-$\alpha_1$-Fragmenten anstelle von Thymosin-$\alpha_1$ in der Immuntherapie Vorteile:

1. Thymosin-$\alpha_1$ ist mit einem Molekulargewicht von 3107 und 28 Aminosäuren ein relativ grosses Polypeptid, dessen Synthese Schwierigkeiten bereitet. Die erfindungsgemässen Thymosin-$\alpha_1$-Fragmente dagegen sind sehr viel kleiner und lassen sich daher wesentlich einfacher, mit höheren Ausbeuten und besserer Reinheit herstellen.

2. Man findet unter den Thymosin-$\alpha_1$-Fragmenten sowohl Peptide mit immunstimulierender als auch Peptide mit immunsuppressiver Wirkung. Bei den Fragmenten mit immunstimulierender Wirkung handelt es sich hauptsächlich um C-terminale Fragmente, während den N-terminalen Fragmenten eher eine immunsuppressive Wirkung zukommt. Entsprechend ihrer unterschiedlichen pharmakologischen Eigenschaften lassen sich diese Fragmente einzeln oder in Kombination zu einer gezielten Immuntherapie einsetzen.

Es wurden nun weitere Fragmente des Thymosin-$\alpha_1$ gefunden, die überraschenderweise auch die Eigenschaften der schon bekannten Fragmente besitzen.

Gegenstand der Erfindung ist daher ein Arzneimittel mit immunregulierender Wirkung, welches mindestens 1 Thymosin-$\alpha_1$-Fragment und/oder mindestens ein Derivat davon in freier Form oder in Form eines pharmakologisch annehmbaren Salzes enthält, welches durch die Formel $R_1$-Val-Val-$R_2$ gekennzeichnet ist, wobei $R_1$=H-, Glu-, Lys-Lys-Glu-, Glu-Lys-Lys-Glu-, Lys-Glu-Lys-Lys-Glu oder Thr-Lys-Asp-Leu-Lys-Glu-Lys-Lys-Glu ist und $R_2$= -H oder -Glu ist.

Ein weiterer Gegenstand der Erfindung ist ein Arzneimittel, welches dadurch gekennzeichnet ist, dass es als Thymosin-$\alpha_1$-Fragment $R_3$-Lys-Glu-$R_4$ enthält, wobei $R_3$=H, Lys, Glu-Lys, Lys-Glu-Lys, Leu-, Asp-Leu- oder Lys-Asp-Leu ist und $R_4$= -H, -Val, -Val-Val, -Val-Val-Glu-Glu, -Val-Val-Glu-Glu-Ala oder -Val-Val-Glu-Glu-Ala-Glu ist.

Ein weiterer Gegenstand der Erfindung ist ein Arzneimittel, welches dadurch gekennzeichnet ist, dass es als Thymosin-$\alpha_1$-Fragment $R_5$-Glu-Lys-$R_6$ enthält, wobei $R_5$=H-, Lys, Leu-Lys-, Asp-Leu-Lys, Lys-Asp-Leu-Lys- ist und $R_6$= -H ist.

Ein weiterer Gegenstand der Erfindung ist ein Arzneimittel, welches dadurch gekennzeichnet ist, dass es als Thymosin-$\alpha_1$-Fragment $R_7$-Glu-Ala-$R_8$ enthält, wobei $R_7$=H-, Glu- oder Val-Glu- ist und $R_8$=-H oder -Glu ist.

Ein weiterer Gegenstand der Erfindung ist ein Arzneimittel, welches dadurch gekennzeichnet ist, dass es als Thymosin-$\alpha_1$-Fragment $R_9$-Glu-Ile-Thr enthält, wobei $R_9$=H-, Ser- oder Ser-Ser- ist.

Weiterer Gegenstand der Erfindung ist ein Arzneimittel, welches dadurch gekennzeichnet ist, dass es als Thymosin-$\alpha_1$-Fragment $R_{10}$-Ala-Val-Asp enthält, wobei $R_{10}$=H-, Ala- oder Asp-Ala- ist.

Weiterer Gegenstand der Erfindung ist ein Arzneimittel, welches dadurch gekennzeichnet ist, dass es als Thymosin-$\alpha_1$-Fragment Thr-Ser-Ser-Glu-Ile-Thr-Thr-Lys-Asp-Leu-Lys-Glu-Lys enthält.

Die entsprechenden Thymosin-$\alpha_1$-Fragmente sind ebenfalls Gegenstand der Erfindung, mit folgender Ausnahme. In den Fragmenten $R_1$-Val-Val-$R_2$, $R_5$-Glu-Lys-$R_6$ und $R_7$-Glu-Ala-$R_8$ sind die zugehörigen Reste $R_1$ und $R_2$, $R_5$ und $R_6$ bzw. $R_7$ und $R_8$ nicht gleichzeitig H.

Die erfindungsgemässen Arzneimittel können auch zwei oder mehrere der aufgeführten Thymosin-$\alpha_1$-Fragmente enthalten. Beispiele für die erfindungsgemässen Thymosin-$\alpha_1$-Fragmente sind im einzelnen in Tabelle 1 zusammengestellt. Die Fragmente können auch in Form von Derivaten verwendet werden. Bevorzugt werden Derivate, bei denen das N-terminale Ende des Thymosin-$\alpha_1$-Fragments als zusätzliche Aminosäure Acetylserin, Acetyldehydroalanin oder Acetyllysin enthält. Thymosin-$\alpha_1$-Derivate, an deren N-terminalem Ende kein Lysin steht, können als zusätzliche Aminosäure am N-terminalen Ende auch Lysin tragen. Die erfindungsgemässen Thymosin-$\alpha_1$-Frag-

mente können auch in üblicher Weise unter Anwendung von vorzugsweise pharmakologisch annehmbaren Säuren oder Basen in die entsprechenden Salze überführt werden. Neben den Wirkstoffen in Form von Thymosin-$\alpha_1$-Fragmenten und/oder ihren Derivaten können die erfindungsgemässen Arzneimittel auch übliche, für den gewählten Verabreichungsweg geeignete pharmakologisch annehmbare Bindemittel, Trägermaterialien und sonstige Hilfsstoffe enthalten.

Die erfindungsgemässen Thymosin-$\alpha_1$-Fragmente können unter Anwendung üblicher Peptidsyntheseverfahren hergestellt werden, wie durch konventionelle Synthese in Lösung mit maximalem Schutz oder minimalem Schutz der Seitenketten, durch Festphasensynthese nach Merryfield, wobei das Peptid am polymeren Träger aufgebaut wird, oder nach der Polymerreagens-Methode, nach der das Peptid zur Kettenverlängerung über die polymergebundene aktivierte Aminosäure geleitet wird.

In besonders bevorzugter Weise bildet man jedoch die erfindungsgemässen Thymosin-$\alpha_1$-Fragmente nach der Verfahrensweise der deutschen Offenlegungsschrift 2919592 der Anmelderin. Nach dieser Verfahrensweise werden die erfindungsgemässen Thymosin-$\alpha_1$-Fragmente durch schrittweise Sequenzverlängerung mit Hilfe der gemischten Anhydridmethode aufgebaut. Dabei werden insbesondere Schutzgruppen mit tertiärem Kohlenstoffatom in der Urethangruppierung, wie die tertiäre Butyloxycarbonylgruppe, die α,α-Dimethyl-3,5-dimethoxybenzyloxy-carbonylgruppe und die 2-(p-Biphenyl)-propyl-2-oxycarbonylgruppe eingesetzt, die Nebenreaktionen durch sterische Hinderung unterdrücken und es ermöglichen, grössere Überschüsse des gemischten Anhydrids wiederholt anzuwenden.

Die folgenden Beispiele verdeutlichen die Herstellung der erfindungsgemässen Thymosin-$\alpha_1$-Fragmente und ihre pharmakologische Wirksamkeit.

Die verwendeten Abkürzungen entsprechen den IUPAC-IUP-Vorschlägen („J. Biol. Chem.", *247* (1972), 977-983). Dabei bedeuten:

Ddz: α,α-Dimethyl-3,5-dimethoxybenzyloxycarbonyl,
Z: Benzyloxycarbonyl,
Ac: Acetyl,
OBu$^t$: tert.-Butylester,
Mbh: 4,4'-Dimethyloxybenzhydryl,
Me: Methyl,
OBzl: Benzylester, und
Bu$^t$: tert.-Butyl.

Die in den folgenden Beispielen angegebenen Herstellungsmethoden enden bei den geschützten Thymosin-$\alpha_1$-Fragmenten, deren Schutzgruppen wie folgt abgespalten werden können:

a) *Abspalten der Ddz-Schutzgruppe*

Der Ddz-geschützte Peptidester wird mit dem 8- bis 10fachen Überschuss wasserfreier Trifluoressigsäure in Form einer 5%igen Lösung in Methylenchlorid (absolut) versetzt und während 15 bis 30 Minuten bei Raumtemperatur gerührt. Bei längeren Peptiden ist die Zeit zu verlängern. Ist die säurelabile 4,4'-Dimethoxybenzhydrylgruppe in der Sequenz enthalten, so wird die Ddz-Schutzgruppe mit 2,5%iger oder 1%iger Trifluoressigsäure während 30 bis 60 Minuten abgespalten. Zum Reaktionsabbruch wird die Lösung auf −15° C gekühlt und mit der berechneten Menge N-Methylmorpholin (+10%) auf einen pH-Wert von 7,0 bis 7,5 gebracht.

Mit Trifluoressigsäure in Form einer 50%igen Lösung in Methylenchlorid unter Zusatz von Anisol werden primär die tert.-Butylester-Reste sowie die tert.-Butyläther-Gruppen abgespalten, während schliesslich dann die 4,4'-Dimethoxybenzhydril-Schutzgruppe mit 95% Trifluoressigsäure/Anisol abgelöst wird. Enthalten die Fragmente Benzyloxycarbonyl-Schutzgruppen und/oder Benzylester, so werden diese vor der erwähnten Trifluoressigsäurebehandlung in üblicher Weise durch hydrieren in Gegenwart von Palladium auf Kohle in alkoholischer Lösung abgespalten.

*Beispiel 1*

Herstellung von allseits geschützten (19 bis 24), Ddz-Lys(Z)-Lys(Z)-Glu(OBu$^t$)-Val-Val-Glu-(OBu$^t$)-OMe.

*Reaktionslösung A*

Zuerst wurde Ddz-(20-24)-OMe dargestellt.

a) *Ddz-(24)-OMe, Ddz-Glu(OBu$^t$)-OMe*

20 mMol Ddz-Glu(OBu$^t$)-OH wurden in Aceton gelöst und mit der äquivalenten Menge Diazomethan (ätherische Lösung) versetzt. 100% Ausbeute eines gelblichen Öls, chromatographisch einheitlich.

b) *Ddz-(23-24)-OMe, Ddz-Val-Glu(OBu$^t$)-OMe*

7,5 mMol Ddz-Val-OH wurden mit 5 mMol Ddz-Glu(OBu$^t$)-OMe zum Dipeptid umgesetzt. Endreinigung über Kieselgel mit $CHCl_3$/Äthanol 93:7. Das Peptid enthielt noch Ddz-Spaltstück, welches weitere Syntheseschritte nicht beeinflusste.

c) *Ddz-(22-24)-OMe, Ddz-Val-Val-Glu(O-Bu$^t$)-OMe*

Das Tripeptid wurde auf üblichem Wege nach Chromatographie über Sephadex® LH 20 mit Methanol rein erhalten. Es war gut in Methanol und $CHCl_3$ löslich. 60% Ausbeute eines farblosen Öls.

d) *Ddz-(21-24)-OMe, Ddz-Glu(OBu$^t$)-Val-Val-Glu(Obu$^t$)-OMe*

6 mMol Ddz-Glu(OBu$^t$)-OH wurden mit 2,98 mMol Ddz-(22-24)-OMe wie üblich zum Tetrapeptid umgesetzt. Es wurde über eine Sephadex® LH 20/Methanol-Säule gegeben, um in 94%iger Ausbeute das Produkt als farbloses Glas zu erhalten. Das Tetrapeptid löste sich gut in $CHCl_3$, Methanol, Dimethylformamid und Dioxan. Es ist dünnschichtchromatographisch in 93:7,7:1 und 85:10:5 einheitlich und zeigte mit Ninhydrin die gewöhnliche Violette Farbe.

e) *Ddz-(20-24)-OMe, Ddz-Lys(Z)-Glu (OBu$^t$)-Val-Val-Glu(OBu$^t$)-OMe*

9 mMol Ddz-Lys (Z)-OH wurden mit 2,43 mMol Ddz-(21-24)-OMe wie üblich zum Pentapeptid umgesetzt. Nach dem Ausschüttelvorgang wurde über eine Methanol/Sephadex® LH 20-Säule chromatographisch gereinigt, um in 99%iger Ausbeute das Produkt als farbloses Glas zu erhalten.

1,0 g Ddz-(20-24)-OMe wird in 14 ml Dichlormethan gelöst, dem 0,7 ml Trifluoressigsäure zugesetzt waren, um die Ddz-Schutzgruppe abzuspalten. Nach 30 Minuten bei 20° C wurde mit 1,03 ml N-Methylmorpholin neutralisiert. Das dabei gebildete Gel wurde durch Zusatz von 5 ml Dimethylformamid wieder aufgelöst.

*Reaktionslösung B*

693 mg Ddz-Lys-(Z)-OH wurden in 15 ml Dichlormethan unter wasserfreien Bedingungen gelöst, auf −15° C abgekühlt und mit 152 µl N-Methylmorpholin und 162 µl Isobutyloxycarbonylchlorid unter heftigem Rühren versetzt. Nach 8 Minuten Reaktion bei −15° C wurde die obige Lösung A zugegeben und ohne Kühlung noch 3 Stunden gerührt.

Zur Aufarbeitung wurde das Reaktionsgemisch mit 100 ml Chloroform verdünnt, bei 0° C mit 0,5 M $KHSO_4$-Lösung, 5% $KHCO_3$-Lösung und Wasser extrahiert und im Vakuum bei 30° C eingedampft.

Nach Kristallisation aus Äthylacetat/Äther, Ausbeute 1,125 g (91% der Theorie), Fp. 216-218° C (Zers.).

Aminosäurenanalyse: Glu 2,20(2), Val 1,94(2), Lys 2,00(2). Dünnschichtchromatographie (Kieselgel Merck $F_{254}$, 0,250 mm).

| $R_f$×100 | Laufmittel |
|---|---|
| 73 | $CHCl_3/CH_3OH/CH_3CO_2H$ 85:10:5 (V/V) |
| 60 | $CHCl_3/C_2H_5OH$ 9:1 (V/V) |

*Beispiel 2*

Herstellung von allseits geschütztem [18-24], Ddz-Glu(OBu$^t$)-Lys(Z)-Lys(Z)-Glu(OBu$^t$)-Val-Val-Glu(OBu$^t$)OMe.

735 mg Ddz[19-24]OMe, erhalten nach Beispiel 1, werden entsprechend der voranstehenden Prozedur A von der Ddz- Schutzgruppe befreit und mit der Reaktionslösung entsprechend Prozedur B von 787 mg Ddz-Glu(OBu$^t$)CHA (CHA=Cyclohexylammoniumsalz) und 97 µl Isobutyloxy- carbonylchlorid 5 Stunden umgesetzt und wie dort aufgearbeitet.

Nach Kristallisation aus Äthylacetat, Ausbeute 730 mg (87% der Theorie) Aminosäurenanalyse: Glu 2,99(3), Val 2,00(2), Lys 1,91(2).

| $R_f$×100 | Laufmittel |
|---|---|
| 69 | $CHCl_3/CH_3OH/CH_3CO_2H$ 85:10:5 (V/V) |
| 59 | $CHCl_3/CH_3OH$ 9:1 (V/V) |

*Beispiel 3*

Herstellung von allseits geschütztem [17-24], Ddz-Lys(Z)-Glu(OBu$^t$)-Lys(Z)-Glu(OBu$^t$)-Val-Val-Glu(OBu$^t$)OMe.

350 mg Ddz[18-24]OMe, erhalten nach Beispiel 2, werden entsprechend der Prozedur A von der Ddz-Schutzgruppe befreit und mit der Reaktionslösung entsprechend Prozedur B von 186 mg Ddz-Lys(Z)OH und 43,2 µl Isobutyloxycarbonylchlorid 5 Stunden umgesetzt und wie dort aufgearbeitet.

Nach zweimaligem Umkristallisieren aus siedendem Methanol, Ausbeute 245 mg (60% der Theorie), Aminosäurenanalyse: Glu 3,38(3), Val 2,00(2), Lys 2,80(3).

| $R_f$×100 | Laufmittel |
|---|---|
| 67 | $CHCl_3/CH_3OH/CH_3CO_2H$ 85:10:5 (V/V) |

*Beispiel 4*

Herstellung von allseits geschütztem [25-27], Ddz-Glu(OBu$^t$)-Ala-Glu(OBu$^t$)OBu$^t$.

1,5 g Ddz-Ala-Glu(OBu$^t$)OBu$^t$ werden entsprechend der Prozedur A von der Ddz-Schutzgruppe befreit und mit der Reaktionslösung entsprechend Prozedur B von 2,84 g Ddz-Glu(OBu$^t$)CHA und 0,62 ml Isobutyloxycarbonylchlorid 2 Stunden umgesetzt. Nach der Aufarbeitung wie dort wurde an Sephadex® LH 20 in Methanol chromatographisch gereinigt und aus Äther/Benzin (40° C) kristallisiert.

Ausbeute 1,7 g (85% der Theorie), Fp. 88 bis 90° C.

Aminosäureanalyse: Glu 1,70(2), Ala 1,00(1).

| $R_f$×100 | Laufmittel |
|---|---|
| 45 | $CHCl_3/CH_3COCH_3$ 85:15 (V/V) |

*Beispiel 5*

Herstellung von allseits geschütztem [20-25], Ddz-Lys(Z)-Glu(OBu$^t$)-Val-Val-Glu(OBu$^t$)Glu (OBu$^t$)OBu$^t$. 250 mg Ddz-/20-24/OH, hergestellt nach Beispiel 1, und 73,5 mg 1-Hydroxybenzotriazol (HOBT) werden in 20 ml absolutem Tetrahydrofuran gelöst und bei 0° C mit 50 mg Dicyclohexylcarbodiimid, gefolgt von 65 mg HGLu(OBu$^t$)OBu$^t$, umgesetzt während 2 Stunden Reaktionszeit bei 0° C und 4 Stunden bei 20° C. Nach dem Eindampfen im Vakuum bei 30° C wird in 50 ml Äthylacetat aufgenommen, Dicyclohexylharnstoff abfiltriert und das Filtrat mit 0,5 M $KHSO_4$-Lösung, 5%iger $KHCO_3$-Lösung und Wasser extrahiert. Die organische Phase wird über bas. $Al_2O_3$ (Säule 3×10 cm) filtriert, die mit 250 ml Äthylacetat nachgewaschen wird. Das Eluat wird im Vakuum eingedampft bei 30° C und hinterlässt das Endprodukt als farbloses Glas in reiner Form. Ausbeute 160 mg (52% der Theorie).

Aminosäurenanalyse: Glu 2,90(3), Val 2,00(2), Lys 0,99(1).

| $R_f$×100 | Laufmittel |
|---|---|
| 91 | $CHCl_3/CH_3OH/CH_3CO_2H$ 85:10:5 (V/V) |
| 58 | $CHCl_3/C_2H_5OH$ 9:1 (V/V) |

*Beispiel 6*

Herstellung von allseits geschütztem [25-26], Ddz-Glu(OBu$^t$)-AlaOBzl.

Entsprechend der Prozedur B werden 2,1 g Ddz-Glu(OBu$^t$)CHA mit 468 µl Isobutyloxycarbonylchlorid aktiviert und mit 500 mg HCl·AlaOBzl und 443 µl N-Methylmorpholin umgesetzt. Nach Eindampfen bei 30° C im Vakuum wurde der ölige Rückstand in 5 ml Äthylacetat aufgenommen und über eine Säule (3×10 cm) mit bas. $Al_2O_3$ filtriert.

Nach Elution mit 250 ml Äthylacetat und Eindampfen im Vakuum bei 30° C wurde das reine Produkt als farbloses Glas gewonnen.

Ausbeute: 1,31 g (96% der Theorie).

| $R_f$×100 | Laufmittel |
|---|---|
| 85 | $CHCl_3/CH_3OH/CH_3CO_2H$ 85:10:5 (V/V) |
| 51 | $CHCl_3/CH_3COCH_3$ 85:15 (V/V) |

*Beispiel 7*

Herstellung von allseits geschütztem [20-26], Ddz-Lys(Z)-Glu(OBu$^t$)-Val-Val-Glu(OBu$^t$)Glu(OBu$^t$)-AlaOBzl.

Entsprechend der Prozedur A wurde von 176 mg Ddz[25-26]OBzl, hergestellt nach Beispiel 6, die Ddz-Schutzgruppe abgespalten, mit 330 µl neutralisiert und im Vakuum bei 20° C eingedampft. Der Rückstand wurde in 2 ml Dimethylformamid gelöst und dem nachfolgend beschriebenen Ansatz zugesetzt.

250 mg Ddz[20-24]OH und 73,5 mg HOBT wurden in 3 ml Dimethylformamid gelöst, bei 0° C mit 100 mg Dicyclohexylcarbodiimid aktiviert und mit der obigen Lösung von H[25-26]Bzl versetzt. Nach Reaktion von 1 Stunde bei 0° C und 3 Tagen bei 20° C wurde entsprechend der Synthese von Fragment [20-25] aufgearbeitet und gereinigt.

Nach Umkristallisation aus Methanol, Ausbeute 190 mg (58% der Theorie).

Aminosäureanalyse: Glu 3,06(3), Ala 1,00(1), Val 1,77(2), Lys 1,05(1).

| $R_f$×100 | Laufmittel |
|---|---|
| 78 | $CHCl_3/C_2H_5OH$ 9:1 (V/V) |

Für eine Reihe von erfindungsgemässen Thymosin-$\alpha_1$-Fragmenten sind in der nachstehenden Tabelle 2 die $R_f$-Werte angegeben. Sie wurden auf Kieselgeldünnschichtplatten, 0,25 mm, MERCK 60 $F_{254}$ bestimmt und sind als $R_f$×100-Werte wiedergegeben.

Laufmittel 1: n-Butanol/Pyridin/Eisessig/Wasser 5:5:1:4 (V/V)
Laufmittel 2: Essigester/Pyridin/Eisessig/Wasser 15:20:6:11 (V/V)
Laufmittel 3: n-Pentanol/Pyridin/2-Butanon/Ameisensäure/Wasser 40:28:11:5:15 (V/V)

Die Bezeichnung der Fragmente erfolgt in der Nummerierung von Tabelle 1. ⟶

*Beispiel 8*

Zur Verdeutlichung der pharmakologischen Wirksamkeit der erfindungsgemässen Thymosin-$\alpha_1$-Fragmente wurden pharmakologische Vergleichsversuche durchgeführt, bei denen die erfindungsgemässen Fragmente einerseits einem synthetischen Thymosin-$\alpha_1$ und anderseits der be-

⟶ *Tabelle 1*

*Thymosin-$\alpha_1$-Fragmente mit immunregulierender Wirkung*

| Fragment Nr. | Fragmente der Formel | |
|---|---|---|
| | | $R_1$-Val-Val-$R_1$ |
| 1 | 22-24 | Val-Val-Glu |
| 2 | 21-23 | Glu-Val-Val |
| 3 | 21-24 | Glu-Val-Val-Glu |
| 4 | 19-24 | Lys-Lys-Glu-Val-Val-Glu |
| 5 | 18-24 | Glu-Lys-Lys-Glu-Val-Val-Glu |
| 6 | 17-24 | Lys-Glu-Lys-Lys-Glu-Val-Val-Glu |
| 7 | 13-24 | Thr-Lys-Asp-Leu-Lys-Glu-Lys-Lys-Glu-Val-Val-Glu |
| | | $R_1$-Lys-Glu-$R_2$ |
| 8 | 17-18 | Lys-Glu |
| 9 | 20-22 | Lys-Glu-Val |
| 10 | 20-23 | Lys-Glu-Val-Val |
| 11 | 20-25 | Lys-Glu-Val-Val-Glu-Glu |
| 12 | 20-26 | Lys-Glu-Val-Val-Glu-Glu-Ala |
| 13 | 20-27 | Lys-Glu-Val-Val-Glu-Glu-Ala-Glu |
| 14 | 19-22 | Lys-Lys-Glu-Val |
| 15 | 18-22 | Glu-Lys-Lys-Glu-Val |
| 16 | 17-22 | Lys-Glu-Lys-Lys-Glu-Val |
| 17 | 16-22 | Leu-Lys-Glu-Lys-Lys-Glu-Val |
| 18 | 16-18 | Leu-Lys-Glu |
| 19 | 15-18 | Asp-Leu-Lys-Glu |
| 20 | 14-18 | Lys-Asp-Leu-Lys-Glu |
| | | $R_1$-Glu-Lys-$R_2$ |
| 22 | 17-19 | Lys-Glu-Lys |
| 23 | 16-19 | Leu-Lys-Glu-Lys |
| 24 | 15-19 | Asp-Leu-Lys-Glu-Lys |
| 25 | 14-19 | Lys-Asp-Leu-Lys-Glu-Lys |
| 26 | 7-19 | Thr-Ser-Ser-Glu-Ile-Thr-Thr-Lys-Asp-Leu-Lys-Glu-Lys |
| | | $R_1$-Glu-Ala-$R_2$ |
| 28 | 25-27 | Glu-Ala-Glu |
| 29 | 24-26 | Glu-Glu-Ala |
| 30 | 23-26 | Val-Glu-Glu-Ala |
| | | R-Glu-Ile-Thr |
| 31 | 10-12 | Glu-Ile-Thr |
| 32 | 9-12 | Ser-Glu-Ile-Zhr |
| 33 | 8-12 | Ser-Ser-Glu-Ile-Thr |

| Fragment Nr. | Fragmente der Formel $R_1$-Val-Val-$R_1$ | |
|---|---|---|
| | R-Ala-Val-Asp | |
| 34 | 4-6 | Ala-Val-Asp |
| 35 | 3-6 | Ala-Ala-Val-Asp |
| 36 | 2-6 | Asp-Ala-Ala-Val-Asp |

*Tabelle 2*

| Fragment Nr. | $R_f$-Wert Laufmittel | | |
|---|---|---|---|
| | 1 | 2 | 3 |
| 12 | 40 | 61 | 28 |
| 13 | 14 | 17 | 3 |
| 14 | 14 | 18 | 2 |
| 15 | 6 | 9 | 0 |
| 20 | 19 | 29 | 5 |
| 22 | 18 | 25 | 3 |

kannten Thymosinfraktion Nr. 5 gegenübergestellt wurden. Bei diesen pharmakologischen Vergleichsversuchen wurden die Methode der gemischten Lymphozytenkultur einerseits und der E-Rosettentest andererseits durchgeführt, wobei beide Testmethoden mit zusätzlicher Inhibierung der zellulären RNS-Polymerase durch α-Amanitin ausgeführt wurden. Hierbei wurden im einzelnen die folgenden Bedingungen angewandt:

1. Gemische Lymphozytenkultur (MLC), α-Amanitin-Inhibierung

Man suspendiert menschliche periphere T-Lymphozyten von zwei gesunden Spendern ($10^5$ T-Zellen) in 100 µl eines Lymphozytenkulturmediums und inhibiert diese durch Zugabe von 2 µg α-Amanitin in 20 µl des Mediums. Anschliessend gibt man 0,004 µg der zu untersuchenden Probe in 20 µl des Mediums zu (Responderzellen-Präparat). Zu dieser Kultur gibt man $10^5$ T-Zellen, die getrennt mit Mytomycin-C blockiert worden sind, in Form einer Suspension in 100 µl des Mediums (Stimulatorzellen-Präparat, wie es üblicherweise bei der MCL verwendet wird).

Man inkubiert die Kultur (mit einem Gesamtvolumen von 240 µl) während 120 Stunden bei 37° C. Während der letzten acht Inkubierungsstunden gibt man 100 µ Ci $^3$H-Thymidin zu der Kultur zu. Nach dem Isolieren der Zellen bestimmt man die $^3$H-Thymidin-Aufnahme durch Flüssigszintillationszählung. Sämtliche Bestimmungen werden mindestens dreifach in einem Doppelblindversuch durchgeführt.

Bewertung: Bei sämtlichen MLC-Kulturen werden vorher oder nachher α-amanitin-inhibierte Blindversuche $B_1$ und $B_2$ durchgeführt, wonach man den Mittelwert bildet: $B=(B_1+B_2)/2$. Sämtliche MLC-Bestimmungen werden in drei Gruppen in Abhängigkeit von der Durchführungszeit eingeteilt. Die Berechnung der ersten Gruppe wird auf den Blindversuch $B_1$ bezogen, die mittlere Gruppe wird auf B bezogen und die letzte Gruppe wird mit dem Blindversuch $B_2$ verglichen. Das stärkste Ansprechen erzielt man mit dem synthetischen Thymosin-$\alpha_1$, welcher Wert als 100% angenommen wurde. Sämtliche anderen Ergebnisse sind auf diesen relativen prozentualen Massstab bezogen.

2. E-Rosettentest, α-Amanitin-Inhibierung

Bei diesem E-Rosettentest, der nach O. Brodner durchgeführt wird, suspendiert man $20\times10^6$ T-Zellen einer menschlichen peripheren T-Lymphozytenpopulation eines einzigen gesunden Spenders in 4 ml der Hanks-Lösung, wonach man 10 µg α-Amanitin in 1 ml der Hanks-Lösung zusetzt. Man teilt den Pool in 50 Portionen zu 100 µl auf. Zu jedem 100 µl-Anteil gibt man (wärmestabilisiertes) menschliches AB-Serum. Dann inkubiert man die Kulturen über Nacht (13,5 Stunden) bei 20° C, wonach man die Testproben zugibt (2 µg/100 µl Hanks-Pufferlösung) und während 1 Stunde bei 20° C inkubiert. Dann gibt man $5\times10^7$ rote Schafsblutzellen in 100 µl der Hanks-Pufferlösung zu und inkubiert während 5 Min bei 37° C. Dann kühlt man in Eis auf 4° C und zentrifugiert während 5 Min bei 4° C und 100 g. Anschliessend inkubiert man während weiterer 30 Min bei 0° C, wonach man die E-Rosetten auszählt (Aggregate — 3 rote Schafsblutzellen/1 T-Zelle).

Bewertung: Es werden 6 α-amanitin-inhibierte Blindproben durchgeführt (Gesamtvolumen ebenfalls 210 µl), woraus man den Mittelwert der geringen Aktivität ermittelt, der in der relativen Skala als 0% gestzt wird. In dem relativen Massstab wird die mittlere Aktivität des E-Rosettentests ohne Inhibierung mit α-Amanitin und ohne zusätzliche Probe als 100% angesetzt. Sämtliche Bestimmungen wurden nach der Doppelblindversuchtechnik durchgeführt.

Die bei diesen pharmakologischen Untersuchungen ermittelten Ergebnisse sind in der folgenden Tabelle zusammengestellt:

*Tabelle*

*Ergebnisse der pharmakologischen Vergleichsuntersuchungen*

| Untersuchte Probe | MLC-Test relative Aktivität (%) | E-Rosettentest, relative Aktivität (%) |
|---|---|---|
| Nr. 3 (21-24) | 49 | 35 |
| Nr. 4 (19-24) | 64 | 15 |
| Nr. 5 (18-24) | 60 | 53 |
| Nr. 6 (17-24) | 58 | — |
| Nr. 11 (20-25) | 28 | 42 |
| Nr. 12 (20-26) | 90 | 12 |

| Untersuchte Probe | MLC-Test relative Aktivität (%) | E-Rosettentest, relative Aktivität (%) |
|---|---|---|
| Nr. 13 (20-27) | 34 | — |
| Nr. 28 (25-27) | 94 | 35 |
| Nr. 15 (18-22) | 53 | — |
| Nr. 7 (13-24)β | 76 | — |
| Thymosin-$\alpha_1$ | 100 | — |
| Thymosin Frakt. 5 | 40 | 19 |

## Patentansprüche

1. Arzneimittel mit immunregulierender Wirkung, welches mindestens ein Thymosin-$\alpha_1$-Fragment oder/und mindestens ein Derivat davon in freier Form oder in Form eines pharmakologisch annehmbaren Salzes enthält, dadurch gekennzeichnet, dass es als Thymosin-$\alpha_1$-Fragment $R_1$-Val-Val-$R_2$ enthält, wobei
$R_1$=H-, Glu-, Lys-Lys-Glu-, Glu-Lys-Lys-Glu-, Lys-Glu-Lys-Lys-Glu- oder Thr-Lys-Asp-Leu-Lys-Glu-Lys-Lys-Glu- ist und
$R_2$=-H oder -Glu ist,
oder ein Derivat dieses Thymosin-$\alpha_1$-Fragments enthält, dessen N-terminales Ende als zusätzliche Aminosäure Acetylserin, Acetyldehydroalanin, Acetyllysin oder, soweit N-terminal noch kein Lysin steht, Lysin trägt.

2. Arzneimittel nach Anspruch 1, dadurch gekennzeichnet, dass es als Thymosin-$\alpha_1$-Fragment $R_3$-Lys-Glu-$R_4$ enthält, wobei
$R_3$=H-, Lys-, Glu-Lys-, Lys-Glu-Lys-, Leu-, Asp-Leu- oder Lys-Asp-Leu- ist und
$R_4$=-H, -Val, -Val-Val, -Val-Val-Glu-Glu, -Val-Val-Glu-Glu-Ala oder -Val-Val-Glu-Glu-Ala-Glu ist,
oder ein Derivat dieses Thymosin-$\alpha_1$-Fragments enthält, dessen N-terminals Ende als zusätzliche Aminosäure Acetylserin, Acetyldehydroalanin, Acetyllysin oder, soweit N-terminal noch kein Lysin steht, Lysin trägt.

3. Arzneimittel nach Anspruch 1, dadurch gekennzeichnet, dass es als Thymosin-$\alpha_1$-Fragment $R_5$-Glu-Lys-$R_6$ enthält, wobei
$R_5$=H-, Lys-, Leu-Lys-, Asp-Leu-Lys-, Lys-Asp-Leu-Lys- ist und
$R_6$=-H ist,
oder ein Derivat dieses Thymosin-$\alpha_1$-Fragments enthält, dessen N-terminales Ende als zusätzliche Aminosäure, Acetylserin, Acetyldehydroalanin, Acetyllysin oder, soweit N-terminal noch kein Lysin steht, Lysin trägt.

4. Arzneimittel nach Anspruch 1, dadurch gekennzeichnet, dass es als Thymosin-$\alpha_1$-Fragment $R_7$-Glu-Ala-$R_8$ enthält, wobei
$R_7$=H-, Glu- oder Val-Glu- ist und
$R_8$=-H oder -Glu ist,
oder ein Derivat dieses Thymosin-$\alpha_1$-Fragments enthält, dessen N-terminales Ende als zusätzliche Aminosäure Acetylserin, Acetyldehydroalanin, Acetyllysin oder, soweit N-terminal noch kein Lysin steht, Lysin trägt.

5. Arzneimittel nach Anspruch 1, dadurch gekennzeichnet, dass es als Thymosin-$\alpha_1$-Fragment $R_9$-Glu-Ile-Thr enthält, wobei
$R_9$=H-, Ser- oder Ser-Ser- ist,
oder ein Derivat dieses Thymosin-$\alpha_1$-Fragments enthält, dessen N-terminales Ende als zusätzliche Aminosäure Acetylserin, Acetyldehydroalanin, Acetyllysin oder, soweit N-terminal noch kein Lysin steht, Lysin trägt.

6. Arzneimittel nach Anspruch 1, dadurch gekennzeichnet, dass es als Thymosin-$\alpha_1$-Fragment $R_{10}$-Ala-Val-Asp enthält, wobei
$R_{10}$=H-, Ala- oder Asp-Ala- ist,
oder ein Derivat dieses Thymosin-$\alpha_1$-Fragments enthält, dessen N-terminales Ende als zusätzliche Aminosäure Acetylserin, Acetyldehydroalanin, Acetyllysin oder, soweit N-terminal noch kein Lysin steht, Lysin trägt.

7. Arzneimittel nach Anspruch 1, dadurch gekennzeichnet, dass es das Thymosin-$\alpha_1$-Fragment der Formel Thr-Ser-Ser-Glu-Ile-Thr-Thr-Lys-Asp-Leu-Lys-Glu-Lys oder ein Derivat dieses Thymosin-$\alpha_1$-Fragments enthält, dessen N-terminales Ende als zusätzliche Aminosäure Acetylserin, Acetyldehydroalanin, Acetyllysin oder Lysin trägt.

8. Thymosin-$\alpha_1$-Fragment der Formel
$R_1$-Val-Val-$R_2$, in der
$R_1$=H-, Glu-, Lys-Lys-Glu-, Glu-Lys-Lys-Glu-, Lys-Glu-Lys-Lys-Glu- oder Thr-Lys-Asp-Leu-Lys-Glu-Lys-Lys-Glu- und
$R_2$=-H oder -Glu, aber $R_1$ und $R_2$ nicht gleichzeitig H,
bedeuten und dessen Derivate, die am N-terminalen Ende als zusätzliche Aminosäure Acetylserin, Acetyldehydroalanin, Acetyllysin oder, soweit N-terminal noch kein Lysin steht, Lysin tragen.

9. Thymosin-$\alpha_1$-Fragment der Formel
$R_3$-Lys-Glu-$R_4$, in der
$R_3$=H-, Lys-, Glu-Lys-, Lys-Glu-Lys-, Leu-, Asp-Leu- oder Lys-Asp-Leu- und
$R_4$=-H, -Val, -Val-Val, -Val-Val-Glu-Glu, -Val-Val-Glu-Glu-Ala oder -Val-Val-Glu-Glu-Ala-Glu
bedeuten und dessen Derivate, die am N-terminalen Ende als zusätzliche Aminosäure Acetylserin, Acetyldehydroalanin, Acetyllysin oder, soweit N-terminal noch kein Lysin steht, Lysin tragen.

10. Thymosin-$\alpha_1$-Fragment der Formel
$R_5$-Glu-Lys-$R_6$, in der
$R_5$=H-, Lys-, Leu-Lys-, Asp-Leu-Lys-, Lys-Asp-Leu-Lys- und
$R_6$=-H, aber $R_5$ und $R_6$ nicht gleichzeitig H,
bedeuten und dessen Derivate, die am N-terminalen Ende als zusätzliche Aminosäure Acetylserin, Acetyldehydroalanin, Acetyllysin oder, soweit N-terminal noch kein Lysin steht, Lysin tragen.

11. Thymosin-$\alpha_1$-Fragment der Formel
$R_7$-Glu-Ala-$R_8$, in der
$R_7$=H-, Glu- oder Val-Glu- und
$R_8$=-H oder -Glu, aber $R_7$ und $R_8$ nicht gleichzeitig H,
bedeuten und dessen Derivate, die am N-terminalen Ende als zusätzliche Aminosäure Acetylserin,

Acetyldehydroalanin, Acetyllysin oder, soweit N-terminal noch kein Lysin steht, Lysin tragen.

12. Thymosin-$\alpha_1$-Fragment der Formel

$R_9$-Glu-Ile-Thr, in der

$R_9$=H-, Ser- oder Ser-Serbedeuten und dessen Derivate, die am N-terminalen Ende als zusätzliche Aminosäure Acetylserin, Acetyldehydroalanin, Acetyllysin oder, soweit N-terminal noch kein Lysin steht, Lysin tragen.

13. Thymosin-$\alpha_1$-Fragment der Formel

$R_{10}$-Ala-Val-Asp, in der

$R_{10}$=H-, Ala- oder Asp-Alabedeuten und dessen Derivate, die am N-terminalen Ende als zusätzliche Aminosäure Acetylserin, Acetyldehydroalanin, Acetyllysin oder, soweit N-terminal noch kein Lysin steht, Lysin tragen.

14. Thymosin-$\alpha_1$-Fragment der Formel

Thr-Ser-Ser-Glu-Ile-Thr-Thr-Lys-Asp-Leu-Lys-

Glu-Lys und

dessen Derivate, die am N-terminalen Ende als zusätzliche Aminosäure Acetylserin, Acetyldehydroalanin, Acetyllysin oder, soweit N-terminal noch kein Lysin steht, Lysin tragen.

## Claims

1. Medicament with immune-regulating action which contains at least one thymosine $\alpha_1$ fragment and/or at least one derivative thereof in free form of in the form of a pharmacologically acceptable salt, characterised in that as thymosine $\alpha_1$ fragment it contains

$R_1$-Val-Val-$R_2$, whereby

$R_1$=H-, Glu-, Lys-Lys-Glu-, Glu-Lys-Lys-Glu-, Lys-Glu-Lys-Lys-Glu or Thr-Lys-Asp-Leu-Lys-Glu-Lys-Lys-Glu- and

$R_2$=-H or -Glu,

or contains a derivative of this thymosine $\alpha_1$ fragment, the N-terminal end of which carries, as additional amino acid, acetylserine, acetyldehydroalanine, acetyllysine or, insofar as there is no N-terminal lysine, lysine.

2. Medicament according to claim 1, characterised in that, as thymosine $\alpha_1$ fragment, it contains

$R_3$-Lys-Glu-$R_4$, whereby

$R_3$=H-, Lys-, Glu-Lys-, Lys-Glu-Lys-, Leu-, Asp-Leu- or Lys-Asp-Leu- and

$R_4$=-H, -Val, -Val-Val, -Val-Val-Glu-Glu, -Val-Val-Glu-Glu-Ala or -Val-Val-Glu-Glu-Ala-Glu,

or contains a derivative of this thymosine $\alpha_1$ fragment, the N-terminal end of which carries, as additional amino acid, acetylserine, acetyldehydroalanine, acetyllysine or, insofar as there is no N-terminal lysine, lysine.

3. Medicament according to claim 1, characterised in that, as thymosine $\alpha_1$ fragment, it contains $R_5$-Glu-Lys-$R_6$, whereby $R_5$=H-, Lys-, Leu-Lys-, Asp-Leu-Lys-, Lys-Asp-Leu-Lys- and $R_6$=-H, or contains a derivative of this thymosine $\alpha_1$ fragment, the N-terminal end of which carries, as additional amino acid, acetylserine, acetyldehydroalanine, acetyllysine or, insofar as there is no terminal N-lysine, lysine.

4. Medicament according to claim 1, characterised in that, as thymosine $\alpha_1$ fragment, it contains

$R_7$-Glu-Ala-$R_8$, whereby

$R_7$=H-, Glu- or Val-Glu- and

$R_8$=-H or -Glu,

or contains a derivative of this thymosine $\alpha_1$ fragment, the N-terminal end of which carries, as additional amino acid, acetylserine, acetyldehydroalanine, acetyllysine or, insofar as there is no N-terminal lysine, lysine.

5. Medicament according to claim 1, characterised in that, as thymosine $\alpha_1$ fragment, it contains

$R_9$-Glu-Ile-Thr-, whereby

$R_9$=H-, Ser- or Ser-Ser-,

or contains a derivative of this thymosine $\alpha_1$ fragment, the N-terminal end of which carries, as additional amino acid, acetylserine, acetyldehydroalanine, acetyllysine or, insofar as there is no N-terminal lysine, lysine.

6. Medicament according to claim 1, characterised in that, as thymosine $\alpha_1$ fragment, it contains

$R_{10}$-Ala-Val-Asp, whereby

$R_{10}$=H-, Ala- or Asp-Ala-,

or contains a derivative of this thymosine $\alpha_1$ fragment, the N-terminal end of which carries, as additional amino acid, acetylserine, acetyldehydroalanine, acetyllysine or, insofar as there is no N-terminal lysine, lysine.

7. Medicament according to claim 1, characterised in that it contains the thymosine $\alpha_1$ fragment of the formula

Thr-Ser-Ser-Glu-Ile-Thr-Thr-Lys-Asp-Leu-Lys-Glu-Lys

or a derivative of this thymosine $\alpha_1$ fragment, the N-terminal end of which carries, as additional amino acid, acetylserine, acetylhydroalanine, acetyllysine or lysine.

8. Thymosine $\alpha_1$ fragment of the formula

$R_1$-Val-Val-$R_2$, in which

$R_1$=H-, Glu-, Lys-Lys-Glu-, Glu-Lys-Lys-Glu-, Lys-Glu-Lys-Lys-Glu- or Thr-Lys-Asp-Leu-Lys-Glu-Lys-Lys-Glu- and

$R_2$=-H or -Glu but $R_1$ and $R_2$

are not simultaneously H, and its derivatives which, on the N-terminal end, carry, as additional amino acid, acetylserine, acetyldehydroalanine, acetyllysine or, insofar as there is no N-terminal lysine, lysine.

9. Thymosine $\alpha_1$ fragment of the formula

$R_3$-Lys-Glu-$R_4$, in which

$R_3$=H-, Lys-, Glu-Lys-, Lys-Glu-Lys-, Leu-, Asp-Leu- or Lys-Asp-Leu- and

$R_4$=-H, -Val, -Val-Val, -Val-Val-Glu-Glu, -Val-Val-Glu-Glu-Ala or -Val-Val-Glu-Glu-Ala-Glu,

and its derivatives which, on the N-terminal end, carry, as additional amino acid, acetylserine, acetyldehydroalanine, acetyllysine or, insofar as there is no N-terminal lysine, lysine.

10. Thymosine $\alpha_1$ fragment of the formula

$R_5$-Glu-Lys-$R_6$, in which

$R_5$=H-, Lys-, Leu-Lys-, Asp-Leu-Lys, Lys-Asp-Leu-Lys- and

$R_6$=-H but $R_5$ and $R_6$

do not simultaneously signify H, and its derivatives which, on the N-terminal end, carry, as additional amino acid, acetylserine, acetyldehydroalanine, acetyllysine or, insofar as there is no N-terminal lysine, lysine.

11. Thymosine $\alpha_1$ fragment of the formula

$R_7$-Glu-Ala-$R_8$, in which

$R_7$=H-, Glu- or Val-Glu- and $R_8$=-H or -Glu, but $R_7$ and $R_8$

do not simultaneously signify H, and its derivatives which, on the N-terminal end, carry, as additional amino acid, acetylserine, acetyldehydroalanine, acetyllysine or, insofar as there is no N-terminal lysine, lysine.

12. Thymosine $\alpha_1$ fragment of the formula

$R_9$-Glu-Ile-Thr, in which

$R_9$=H-, Ser- or Ser-Ser-,

and its derivatives which on the N-terminal end, carry, as additional amino acid, acetylserine, acetyldehydroalanine, acetyllysine or, insofar as there is no N-terminal lysine, lysine.

13. Thymosine $\alpha_1$ fragment of the formula

$R_{10}$-Ala-Val-Asp, in which

$R_{10}$=H-, Ala- or Asp-Ala-,

and its derivatives which, on the N-terminal end, carry, as additional amino acid, acetylserine, acetyldehydroalanine, acetyllysine or, insofar as there is no N-terminal lysine, lysine.

14. Thymosine $\alpha_1$ fragment of the formula

Thr-Ser-Ser-Glu-Ile-Thr-Thr-Lys-Asp-Leu-Lys-Glu-Lys

and its derivatives which, on the N-terminal end, carry, as additional amino acid, acetylserine, acetyldehydroalanine, acetyllysine or, insofar as there is no N-terminal lysine, lysine.

## Revendications

1. Médicament à action immunorégulatrice contenant au moins un fragment de thymosine $\alpha_1$ et/ou au moins un dérivé d'un tel fragment à l'état libre ou à l'état de sel pharmacologiquement acceptable, caractérisé en ce qu'il contient, en tant que fragment de thymosine $\alpha_1$, le fragment $R_1$-Val-Val-$R_2$ dans lequel

$R_1$=H-, Glu-, Lys-Lys-Glu-, Glu-Lys-Lys-Glu-, Lys-Glu-Lys-Lys-Glu- ou Thr-Lys-Asp-Leu- Lys-Glu-Lys-Lys-Glu- et

$R_2$=-H ou -Glu

ou un dérivé de ce fragment de thymosine $\alpha_1$, dont l'extrémité à azote terminal porte en tant qu'aminoacide supplémentaire l'acétylsérine, l'acétyldéshydroalanine, l'acétyllysine ou bien, lorsque l'azote terminal ne porte pas encore de lysine, la lysine.

2. Médicament selon la revendication 1, caractérisé en ce qu'il contient, en tant que fragment de thymosine $\alpha_1$, le fragment $R_3$-Lys-Glu-$R_4$ dans lequel

$R_3$=H-, Lys-, Glu-Lys-, Lys-Glu-Lys-, Leu-, Asp-Leu- ou Lys-Asp-Leu- et

$R_4$=-H, -Val, -Val-Val, -Val-Val-Glu-Glu, -Val-Val-Glu-Glu-Ala ou -Val-Val-Glu-Glu-Ala-Glu ou un dérivé de ce fragment de thymosine $\alpha_1$, dont l'extrémité à azote terminal porte en tant qu'aminoacide supplémentaire l'acétylsérine, l'acétyldéshydroalanine, l'acétyllysine ou bien, lorsque l'azote terminal ne porte pas encore de lysine, la lysine.

3. Médicament selon la revendication 1, caractérisé en ce qu'il contient, en tant que fragment de thymosine $\alpha_1$, le fragment $R_5$-Glu-Lys-$R_6$ dans lequel

$R_5$=H-, Lys-, Leu-Lys-, Asp-Leu-Lys-, Lys-Asp-Leu-Lys- et

$R_6$=-H,

ou un dérivé de ce fragment de thymosine $\alpha_1$ dont l'extrémité à azote terminal porte en tant qu'aminoacide supplémentaire l'acétylsérine, l'acétyldéshydroalanine, l'acétyllysine ou bien, lorsque l'azote terminal ne porte pas encore de lysine, la lysine.

4. Médicament selon la revendication 1, caractérisé en ce qu'il contient, en tant que fragment de thymosine $\alpha_1$, le fragment $R_7$-Glu-Ala-$R_8$ dans lequel

$R_7$=H-, Glu- ou Val-Glu- et

$R_8$=-H ou -Glu,

ou un dérivé de ce fragment de thymosine $\alpha_1$ dont l'extrémité à azote terminal porte en tant qu'aminoacide supplémentaire l'acétylsérine, l'acétyldéshydroalanine, l'acétyllysine ou bien, lorsque l'azote terminal ne porte pas encore de lysine, la lysine.

5. Médicament selon la revendication 1, caractérisé en ce qu'il contient, en tant que fragment de thymosine $\alpha_1$, le fragment $R_9$-Glu-Ile-Thr dans lequel

$R_9$=H-, Ser- ou Ser-Ser-,

ou un dérivé de ce fragment de thymosine $\alpha_1$ dont l'extrémité à azote terminal porte en tant qu'aminoacide supplémentaire l'acétylsérine, l'acétyldéshydroalanine, l'acétyllysine ou bien, lorsque l'azote terminal ne porte pas encore de lysine, la lysine.

6. Médicament selon la revendication 1, caractérisé en ce qu'il contient, en tant que fragment de thymosine $\alpha_1$, le fragment $R_{10}$-Ala-Val-Asp dans lequel

$R_{10}$=H-, Ala- ou Asp-Ala,

ou un dérivé de ce fragment de thymosine $\alpha_1$ dont l'extrémité à azote terminal porte en tant qu'aminoacide supplémentaire l'acétylsérine, l'acétyldéshydroalanine l'acétyllysine ou bien, lorsque l'azote terminal ne porte pas encore de lysine, la lysine.

7. Médicament selon la revendication 1, caractérisé en ce qu'il contient le fragment de thymosine $\alpha_1$ de formule

Thr-Ser-Ser-Glu-Ile-Thr-Thr-Lys-Asp-Leu-Lys-Glu-Lys

ou un dérivé de ce fragment de thymosine $\alpha_1$ dont l'extrémité à azote terminal porte en tant qu'aminoacide supplémentaire l'acétylsérine, l'acétyldéshydroalanine, l'acétyllysine ou la lysine.

8. Fragment de thymosine $\alpha_1$ de formule

$R_1$-Val-Val-$R_2$ dans laquelle

$R_1$=H-, Glu-, Lys-Lys-Glu-, Glu-Lys-Lys-Glu, Lys-Glu-Lys-Lys-Glu- ou Thr-Lys-Asp-Leu-Lys-Glu-Lys-Lys-Glu- et

$R_2$=-H ou -Glu, mais $R_1$ et $R_2$ ne peuvent représenter simultanément H,

et ses dérivés portant à l'extrémité à azote terminal, en tant qu'aminoacide supplémentaire, l'acétylsérine, l'acétyldéshydroalanine, l'acétyllysine ou bien, lorsque l'azote terminal ne porte pas encore de lysine, la lysine.

9. Fragment de thymosine $\alpha_1$ de formule

$R_3$-Lys-Glu-$R_4$ dans laquelle

$R_3$=H-, Lys-, Glu-Lys-, Lys-Glu-Lys-, Leu-, Asp-Leu- ou Lys-Asp-Leu- et

$R_4$=-H, -Val, -Val-Val, -Val-Val-Glu-Glu, -Val-Val-Glu-Glu-Ala ou -Val-Val-Glu-Glu-Ala-Glu

et ses dérivés portant à l'extrémité à azote terminal, en tant qu'aminoacide supplémentaire, l'acétylsérine, l'acétyldéshydroalanine, l'acétyllysine ou bien, lorsque l'azote terminal ne porte pas encore de lysine, la lysine.

10. Fragment de thymosine $\alpha_1$ de formule

$R_5$-Glu-Lys-$R_6$, dans laquelle

$R_5$=H-, Lys-, Leu-Lys-, Asp-Leu-Lys-, Lys-Asp-Leu-Lys- et

$R_6$=-H, mais $R_5$ et $R_6$ ne peuvent représenter simultanément H, et ses dérivés portant à l'extrémité à azote terminal, en tant qu'aminoacide supplémentaire, l'acétylsérine, l'acétyldéshydroalanine, l'acétyllysine ou bien, lorsque l'azote terminal ne porte pas encore de lysine, la lysine.

11. Fragment de thymosine $\alpha_1$ de formule

$R_7$-Glu-Ala-$R_8$ dans laquelle

$R_7$=H-, Glu- ou Val-Glu- et

$R_8$=-H ou -Glu, mais $R_7$ et $R_8$ ne peuvent représenter simultanément H,

et ses dérivés portant à l'extrémité à azote terminal, en tant qu'aminoacide supplémentaire, l'acétylsérine, l'acétyldéshydroalanine, l'acétyllysine ou bien, lorsque l'azote terminal ne porte pas encore de lysine, la lysine.

12. Fragment de thymosine $\alpha_1$ de formule

$R_9$-Glu-Ile-Thr dans laquelle

$R_9$=H-, Ser- ou Ser-Ser,

et ses dérivés portant à l'extrémité à azote terminal, en tant qu'aminoacide supplémentaire, l'acétylsérine, l'acétyldeshydroalanine, l'acétyllysine ou bien, lorsque l'azote terminal ne porte pas encore de lysine, la lysine.

13. Fragment de thymosine $\alpha_1$ de formule $R_{10}$-Ala-Val-Asp dans laquelle

$R_{10}$=H-, Ala- ou Asp-Ala-,

et ses dérivés portant à l'extrémité à azote terminal, en tant qu'aminoacide supplémentaire, l'acétylsérine, l'acétyldéshydroalanine, l'acétyllysine ou bien, lorsque l'azote terminal ne porte pas encore de lysine, la lysine.

14. Fragment de thymosine $\alpha_1$ de formule

Thr-Ser-Ser-Glu-Ile-Thr-Thr-Lys-Asp-Leu-Lys-Glu-Lys

et ses dérivés portant à l'extrémité à azote terminal en tant qu'aminoacide supplémentaire, l'acétylsérine, l'acétyldéshydroalanine, l'acétyllysine ou bien, lorsque l'azote terminal ne porte pas encore de lysine, la lysine.